# EUROPEAN PATENT SPECIFICATION

(11) **EP 2 639 232 B1**
(45) Date of publication and mention of the grant of the patent: **03.02.2016**
(21) Application number: 11839391.7
(22) Date of filing: 09.11.2011
(51) Int. Cl.: C07D 311/32, C07D 311/40

(54) **METHOD FOR PRODUCING DIHYDROQUERCETIN**
VERFAHREN ZUR HERSTELLUNG VON DIHYDROQUERCETIN
PROCÉDÉ DE PRODUCTION DE DIHYDROQUERCÉTINE

(30) Priority: 11.11.2010 RU 2010146055
(43) Date of publication of application: 18.09.2013
(73) Proprietor: Ostronkov, Vladimir Sergeevich, Amurskaya obl. 630005 (RU); Lashin, Sergej Alekseevich, Amurskaya obl. 630005 (RU)
(72) Inventor: Ostronkov, Vladimir Sergeevich, Amurskaya obl. 630005 (RU); Lashin, Sergej Alekseevich, Amurskaya obl. 630005 (RU)
(74) Representative: Patentanwälte Zellentin & Partner
(86) International application number: PCT/RU2011/000868
(87) International publication number: WO 2012/064229

(56) References cited:
- WO-A1-2010/095969
- RU-C1- 2 308 267
- RU-C1- 2 330 677
- RU-C1- 2 349 330

## Description

The invention relates to the chemical and pharmaceutical industry, in particular to the production of dihydroquercetin (DHQ) possessing a high antioxidant activity.

DHQ belongs to the class of reduced flavonoids and is a member of the group of flavanon-3-oles. According to its chemical structure DHQ is a polyphenol (3,3',4',5,7-pentahydroxy flavanone) and it displays it's antioxidant activity even at concentrations of 3.3 - 0.3 µmol/l, with a full absence of a mutagenic activity in humans. The agent DHQ is a very important P vitamin with antioxidant activity which ensures vital activity in humans, as it is responsible, in particular, for the capacity of living organisms to resist various pathologies and viral diseases. DHQ is used as a capillary-strengthening drug, capillary-protecting drug, and haemorheologic drug. In pharmacology it is recommended for the combined therapy of avitaminosis, ischemic heart disease, and atherosclerosis, it acts positively on the functional state of the liver, contributes to the recovery of the draining functions of the bronchi and the breathing biomechanism, and improves the heart function. It is an ingredient of phyto concentrates for the prevention and treatment of diabetes mellitus, of diseases of the liver and the gall bladder, of the gastrointestinal tract, the prostate, the kidneys and the urinary bladder, as well as cardiovascular diseases. It possesses a wide range of activities: It regulates metabolic processes and can be used for the combined therapy of various diseases, it acts positively on the vital functions of practically all inner organs of the human body. It is used for the prevention and therapy of tumour diseases (including oncological diseases), in the case of HIV infection, autoimmune diseases, chronic viral and bacterial inflammatory processes, and in the case of acquired pathologies.

However, a broad implementation of DHQ is hampered by its high trade value that is due to the limited availability of its basic raw material (grapes, eucalyptus, cherry bass (*Sagura margaritacea*), rose petals etc.), and to the demand of energy and resources, the complexity and also the ecologically problematic technology that is used for its extraction from the vegetable raw material. Alternative raw materials for the production of DHQ are the roots and the butt of the Siberian larch and the Dahurian larch that grow in Siberia and in the Far East, the wood of these trees containing up to 2.5 per chent of flavonoids that represent mainly DHQ, quercetin, dihydrocempherol and naringenine [N.A. Tyukavkina, K.I. Lapteva, S.A. Medvedeva, "Fenol'nye ekstraktivnye veshchestva roda Larix (obzor)" ["Phenol substances extracted from the genus Larix (review)"] from "Khimiya drevesiny" ["The chemistry of wood"], 1973, 13th edition, p. 3 - 17].

All known methods for producing DHQ from larch wood render a purity level of 90 - 97 per cent. The impurities are in general other flavonoid compounds with a related structure. The removal of these impurities is complicated by the fact that the physical and chemical properties (solubility, sorption capacity, etc.) of these compounds are similar to those of DHQ.

The majority of the known methods for producing dihydroquercetin from larch wood is based on its extraction with various solvents, in particular, the extraction with water and aqueous organic mixtures. The main drawback of the known methods is the large quantity of waste water and the high energy consumption for the regeneration of the extraction agent.

Known is a method for producing dihydroquercetin, consisting in the extraction of wet ground larch wood with an organic solvent, namely, ethyl acetate, subsequent concentration by evaporation of the extract, treatment with hot water, filtration of the aqueous solution for removal of impurities and cristallization of the dihydroquercetin from it, wherein the ground wood is saturated with water directly before the extraction, the evaporated extract is degreased with hexane or benzine, and before cristallization impurities are removed from the aqueous dihydroquercetin solution by filtration (Russian patent No. 2158598, published on Nov. 10, 2000).

The drawback of the known method consists in the difficult preparation of the raw material and in a high consumption of the extraction agent.

Known are methods for the isolation of DHQ that comprise its sorption on various sorbents, followed by desorption by means of a suitable eluent.

Known is, for example, a method for producing DHQ by extraction of sawdust of larch wood by using hot (98 - 100°C) water, followed by purification of the cooled extract by means of a polyamide sorbent (Russian patent No. 2000797, published on October 15, 1993).

The drawback of the known method consists in that a relatively expensive polyamide sorbent is needed to be used that is not produced by domestic industry, in the large quantity of waste water, and in the high energy consumption for the heating of the water.

Known is a method for producing dihydroquercetin in a bioflavonoid complex by extraction of ground larch wood with the use of 96.5 per cent ethyl alcohol, followed by re-extraction with propylene glycol (propandiol-1,2), mixing the propylene glycol extract with an aqueous sodium chloride solution, removal of the resin and the essential oils, applying the dihydroquercetin containing raw material to a polyamide sorbent, washing of the sorbent and eluting the bioflavanoid mixture with 96.5 per cent ethyl alcohol, evaporation of the alcohol, cristallization and drying of the product (Russian patent No. 2186097, published on July 27, 2002).

However, this method does not allow for isolation of dihydroquercetin as an individual component.

Known is a method for isolating DHQ, comprising a preliminary treatment of larch wood with boiling water, followed by extraction with an aqueous acetone solution and purification of the water-acetone extract by liquid chromatography with reversed-phase sorbents of the type Spherisorb C2-C18 with a particle size of 5 - 10 µm, using as a mobile phase a 30 - 50 per cent acetone solution in a 0.1 per cent aqueous solution of trifluoroacetic acid, and removal of the acetone by evaporation. The technical effect consists in obtaining a dihydroquercetin of a very high purity level, exceeding 99.5 per cent (Russian patent No. 2114631, published on July 10, 1998).

The drawback of the known method consists in its complexity and in the use of expensive HPLC sorbents , for which reason it cannot be efficiently adapted to a large-scale industrial production of highly purified dihydroquercetin.

Known is a method for producing highly purified dihydroquercetin from dihydroquercetin containing raw material, comprising the preparation of a solution of the dihydroquercetin containing raw material in a polar solvent, at least one chromatography cycle with the application of a sample of the solution obtained to a column filled with a hydrophobic sorbent, and with thermostatic control at 30 - 50°C, elution of the sorbent with a mobile liquid phase, evaporation of the eluate, drying of the product obtained, wherein as a polar solvent an aqueous solution of an aliphatic C₁-C₃ alcohol is used, wherein the application of the sample is effected at a maximum concentration of the alcohol of 20 vol. per cent, and wherein as mobile phase an aqueous solution of the same alcohol is used, with the concentration of the alcohol in the solution exceeding the concentration of the alcohol in the solution used for the application of the sample by at least 3 per cent, but not more than 30 vol. per cent. Preferably the application of the sample on the column is effected with the concentration of the alcohol in the solution ranging from 1 to 20 vol. per cent, the elution being effected with a concentration of the alcohol in the solution of 12 - 30 vol. per cent (Russian patent No. 2349330, published on March 20, 2009). The method provides for the production of DHQ with a purity of 97.1 - 99.5 per cent and a yield of 89 - 93 per cent.

The drawback of the known method consists in the use of a toxic eluent (methyl alcohol), a high energy consumption for the heating and for holding the temperature of the chromatography column at a constant level, and in the fact that the method is not calculated for application on an industrial scale. Furthermore, as a dihydroquercetin containing raw material ready preparations "Lavitol" and "Sibel" are used that contain the DHQ in an amount of 88 - 96 per cent.

The closest prior art to the present method is a method for producing DHQ consisting in the following. From Siberian or Dahurian larch wood the bark is removed, the wood is chopped and dried at 40 - 50°C to a residual humidity of 23 - 27 per cent. The dried wood is ground, and the soluble substances are extracted using a 75 - 85 per cent aqueous solution of ethyl alcohol at a temperature of 45 - 50°C with a ratio of raw material to extraction agent of 1 : (7-10). The extraction agent is then evaporated, and the sawdust is fed to a press for an additional recovery of alcohol. Then the aqueous portion of the extract is cooled to 20 - 25°C over a period of 20 - 30 minutes for the removal of the resinous impurities accompanying the DHQ. To the resin-free aqueous portion of the extract methyl-tert-butyl ether (MTBE) is added in a ratio of 1 : (0.3-0.45), and DHQ is reextracted over 2 - 3 hours. The ether portion of the extract is separated from the aqueous part by sedimentation over a period of 2 - 2.5 hrs. Then the MTBE is evaporated, and the desired product is crystallized from hot water (patent No. 2330677, published on August 10, 2008). The invention gives DHQ with a yield of 2.2 - 2.5 per cent of the mass of absolutely dry wood, with a purity level of 90 - 96 per cent.

The main drawbacks of the known method consist in an insufficient purity level of the DHQ, the complexity and the duration of the process, the large quantity of waste water, and the high energy consumption for the regeneration of the extraction agent. Furthermore, the use of methyl-tert-butyl ether can lead to serious ecological problems.

The technical problem of the present invention consists in simplifying the known method and increasing its ecological compatibility by means of a reduction of the quantity of waste water and energy consumption for the regeneration of the extraction agent, as well as the exclusion of the toxic extraction agent (methyl-tert-butyl ether).

Said technical problem is achieved by the present method that consists in the following.

From Siberian or Dahurian larch wood the bark is removed, the wood is chopped and dried at 40 - 50°C to a residual humidity of 15 - 25 per cent. The dried wood is ground, and the soluble substances are extracted from the sawdust using a 75 per cent aqueous solution of ethyl alcohol at a temperature of 40 - 50°C with a ratio of raw material to extraction agent of 1 : (6-7). The extraction agent is then distilled off, the sawdust is fed to a screw press for mechanical pressing and for the additional recovery of alcohol. Then the aqueous portion of the extract is cooled to 38 - 42°C over a period of 20 - 30 minutes for the removal of the resinous impurities accompanying the DHQ. The resin-free aqueous portion of the extract having a temperature of 38 - 42°C is fed into a chromatographic column with a diameter of 800 mm and a volume of 300 I, filled with the hydrophobic sorbent LPS-500 on the basis of polydivinylbenzene, with a specific surface area of 700 - 900 m²/g and a microparticle size of 75 - 100 µm. The desired product is eluted with a 32 - 40 per cent aqueous solution of ethyl alcohol. The eluate is collected in a single portion or is divided into two to three fractions, depending upon the required purity of the desired product (DHQ). Each fraction is evaporated until the ethyl alcohol is completely removed, and the residue is crystallized from deionized water. From the first fraction DHQ is obtained with a purity level of 99 - 99.5 per cent, from the second fraction with a purity level of 97 - 98 per cent, and from the third fraction with a purity level of 95 - 96 per cent. The column is cleaned by washing with 96 per cent ethyl alcohol, then filled with water for loading a further portion of raw evaporated aqueous extract. The sorbent in the chromatographic column is used many times, i.e., 300 times or more, without a quality loss.

The method provides for obtaining the desired product with a yield of 1.5 - 2.5 per cent of the mass of absolutely dry wood, and with a purity level of 95.5 - 99.5 per cent.

The determined essential characterizing features of the present method compared to the closest prior art consist in:
- purification of the desired product by high performance liquid chromatography (HPLC) at the hydrophobic sorbent LPS-500 on the basis of polydivinylbenzene, with a specific surface area of 700 - 900 m²/g and a microparticle size of 75 - 100 µm, which feature substantially simplifies the method, reduces its duration and increases the purity of the desired product;
- an aqueous dihydroquercetin extract is fed into the chromatographic column, which feature makes it possible to eliminate the additional step of dissolving in ethyl alcohol, and to reduce the loss of desired product;
- elution is effected with a 32 - 40 per cent solution of ethyl alcohol, which feature allows for a maximum efficiency of separation of the DHQ from the accompanying impurities and similar flavonoids;
- the eluate is collected in a single portion or divided into two or three fractions, each fraction being evaporated until the ethyl alcohol is completely removed, and the residue is crystallized from deionized water, which feature allows to completely eliminate the possibility of the presence of a residue of the solvent ethyl alcohol, and to obtain the desired product with various purity levels, depending on the needs of the user of the DHQ (the cosmetic, food, or pharmaceutical industry).

Consequently, the desired product is obtained on an industrial scale with a purity of 95.5 - 99.5 per cent of its main component, DHQ.

The invention is illustrated by the following examples for its concrete implementation.

### Example 1

From Siberian or Dahurian larch wood the bark is removed, the wood is chopped and dried at 40°C to a residual humidity of 15 - 17 per cent. The dried wood is ground, and the soluble substances are extracted using a 75 per cent aqueous solution of ethyl alcohol at a temperature of 50°C with a ratio of raw material to extraction agent of 1 : 6. Then the extraction agent is distilled off, the sawdust is fed to a screw press for mechanical pressing and for the additional recovery of alcohol. Then the aqueous portion of the extract is cooled to 38°C over a period of 30 minutes for the removal of the heavy resinous impurities accompanying the DHQ. The resin-free aqueous DHQ extract in a quantity of 150 l, having a temperature of 38°C, is fed into a chromatographic column with a diameter of 800 mm and a volume of 300 l, filled with the hydrophobic sorbent LPS-500 on the basis of polydivinylbenzene, with a specific surface area of 700 m²/g and a microparticle size of 75 µm. The desired product is eluted with a 40 per cent solution of ethyl alcohol. The eluate is collected in a single portion which is evaporated until the ethyl alcohol is completely removed, and the residue is crystallized from deionized water. Then the DHQ is dried at a temperature of 45 - 50°C for 10 - 12 hrs in a nitrogen flow, in order to avoid oxidation, to a humidity of 5 - 7 per cent. The yield of desired product is 2.5 per cent. The DHQ is obtained with a purity level of 96 per cent and in a quantity of 5.5 kg. The column is cleaned by washing with 96 per cent ethyl alcohol, then filled with water for loading a further portion of raw evaporated aqueous extract.

### Example 2

From Siberian or Dahurian larch wood the bark is removed, the wood is chopped and dried at 50°C to a residual humidity of 20 - 25 per cent. The dried wood is ground, and the soluble substances are extracted using a 75 per cent aqueous solution of ethyl alcohol at a temperature of 40°C with a ratio of raw material to extraction agent of 1 : 7. Then the extraction agent is distilled off, the sawdust is fed to a screw press for mechanical pressing and for the additional recovery of alcohol. Then the aqueous portion of the extract is cooled to 42°C over a period of 20 minutes for the removal of the resinous impurities accompanying the DHQ. The resin-free aqueous DHQ extract in a quantity of 150 I, having a temperature of 42°C, is fed into a chromatographic column with a diameter of 800 mm and a volume of 300 l, filled with the hydrophobic sorbent LPS-500 on the basis of polydivinylbenzene, with a specific surface area of 900 m²/g and a microparticle size of 100 µm. After the application of the raw material the column is eluted with alcohol. Elution is effected with a 32 per cent solution of ethyl alcohol. The eluate is divided into three fractions, each of which is evaporated until the ethyl alcohol is completely removed, and the residue is crystallized from deionized water. Then the DHQ is dried at a temperature of 45 - 50°C for 10 - 12 hrs in a nitrogen flow, in order to avoid oxidation, to a residual humidity of 5 - 7 per cent. The yield of desired product is 1.5 per cent. The DHQ is obtained with a purity level of 99.5 per cent and in a quantity of 3.2 kg from the first fraction, with a purity level of 98 per cent and in a quantity of 1.7 kg from the second fraction, and with a purity level of 95.5 per cent and in a quantity of 0.5 kg from the third fraction. The column is cleaned by washing with 750 l of 96 per cent ethyl alcohol, then filled with 300 I of water for loading a further portion of raw evaporated aqueous extract.

The use of the present method provides the following effects:
- increase of the purity of the product obtained to 99.5 per cent, preserving the high yield of DHQ;
- simplification of the method by elimination of the lengthy and labour-intensive operations of repeated extraction of the DHQ for 2 - 3 hrs with the use of methyl-tert-butyl ether and subsequent separation of the ether portion of the extract from the aqueous portion by sedimentation for 2 - 2.5 hrs and evaporation of the methyl-tert-butyl ether;
- increase of the ecological compatibility of the process by elimination of the use of the inflammable and toxic reagent methyl-tert-butyl ether from the process, and also by reduction of the quantity of waste water and energy consumption;
- reduction of the cost of the DHQ obtained by using only one solvent (ethyl alcohol), and by substantially reducing the quantity of equipment required for the production (volumenous equipment for storing, sedimentation, mixing, dissolving);
- elimination of the operation of isolation of an intermediate product with a concentration of less than 88 per cent of the main component DHQ, and of its processing for feeding it into the chromatographic column, thus reducing time and energy consumption.

The present method provides for obtaining DHQ with a high yield and a high purity level, substantial simplification of the technology of the process, reduction of the time required for the process, reduction of the costs of the production, and the possibility of processing large quantities of raw material (hundreds of tons).

## Claims

1. Method for producing dihydroquercetin, comprising extraction of dried and ground larch wood with an aqueous solution of ethyl alcohol with heating, distillation of the ethyl alcohol, and cooling of the aqueous portion of the extract for the removal of oil and resin, **characterized in that** the extraction of the ground larch wood is effected at a ratio of raw material to extraction agent of 1 : (6-7), wherein the aqueous portion of the dihydroquercetin extract is cooled to 38 - 42°C, fed into a chromatographic column filled with a hydrophobic sorbent on the basis of polydivinylbenzene, wherein elution of the desired product is effected with 32 - 40 per cent ethyl alcohol, the eluate is collected in a single portion or is divided into two to three fractions, each fraction is evaporated to dryness, the residue is crystallized from water, and the column is cleaned by washing with 96 per cent ethyl alcohol and then filled with water for loading a further portion of aqueous extract.

2. The method of Claim 1, **characterized in that** the extraction of the ground larch wood is effected using a 75 per cent aqueous solution of ethyl alcohol at a temperature of 40 - 50°C.

3. The method of Claim 1, **characterized in that** as a sorbent LPS-500 is used, having a specific surface area of 700 - 900 m²/g and a microparticle size of 75 - 100 µm.

## Patentansprüche

1. Verfahren zur Herstellung von Dihydroquercetin, umfassend eine Extraktion von getrocknetem und zerkleinertem Lärchenholz mit einer wässrigen Lösung von Ethylalkohol unter Erwärmen, eine Destillation des Ethylalkohols, und Abkühlen des wässrigen Teils des Extrakts zur Entfernung von Öl und Harz, **dadurch gekennzeichnet, dass** die Extraktion des zerkleinerten Lärchenholzes bei einem Verhältnis des Ausgangsmaterials zum Extraktionsmittel von 1 : (6-7) erfolgt, wobei der wässrige Teil des Dihydroquercetinextrakts auf 38 - 42 °C gekühlt wird, auf eine chromatographische Säule gegeben wird, die mit einem hydrophoben Sorptionsmittel auf Basis von Polydivinylbenzen gefüllt ist, wobei die Elution des gewünschten Produkts mit 32 - 40 %igem Ethylalkohol erfolgt, das Eluat in einer einzigen Fraktion oder in zwei bis drei Fraktionen aufgeteilt gesammelt wird, wobei jede Fraktion zur Trockne eingedampft wird, der Rückstand aus dem Wasser auskristallisiert wird, und die Säule durch Waschen mit 96 %igem Ethylalkohol gereinigt und dann mit Wasser gefüllt wird zur Beladung mit einem weiteren Teil des wässrigen Extrakts.

2. Verfahren gemäß Anspruch 1, **dadurch gekennzeichnet, dass** die Extraktion des zerkleinerten Lärchenholzes mit einer 75 %igen wässrigen Lösung von Ethylalkohol bei einer Temperatur von 40 - 50 °C erfolgt.

3. Verfahren gemäß Anspruch 1, **dadurch gekennzeichnet, dass** als Sorptionsmittel LPS-500 mit einer spezifischen Oberfläche von 700 bis 900 m²/g und einer Mikropartikelgröße von 75 - 100 nm eingesetzt wird.

## Revendications

1. Procédé de production de dihydroquercétine, comprenant l'extraction de bois de mélèze séché et broyé avec une solution aqueuse d'alcool éthylique en chauffant, la distillation de l'alcool éthylique, et le refroidissement de la portion aqueuse de l'extrait pour la séparation de l'huile et de la résine, **caractérisé en ce que** l'extraction du bois de mélèze broyé est effectuée à un rapport de la matière première à l'agent d'extraction de 1 : (6-7), dans lequel la portion aqueuse de l'extrait de dihydroquercétine est refroidie à 38 - 42 °C, introduite dans une colonne de chromatographie remplie d'un sorbant hydrophobe sur la base de polydivinylbenzène, dans lequel l'élution du produit souhaité est effectuée avec 32 - 40 pour cent d'alcool éthylique, l'éluat est recueilli en une seule portion ou est divisé en deux ou trois fractions, chaque fraction est évaporée à siccité, le résidu est cristallisé dans l'eau, et la colonne est nettoyée par lavage avec 96 pour cent d'alcool éthylique et est ensuite remplie d'eau en perspective du chargement d'une portion d'extrait aqueux supplémentaire.

2. Procédé selon la revendication 1, **caractérisé en ce que** l'extraction du bois de mélèze broyé est effectuée à l'aide d'une solution aqueuse à 75 pour cent d'alcool éthylique à une température de 40 - 50 °C.

3. Procédé selon la revendication 1, **caractérisé en ce qu'**en tant que sorbant, on utilise du LPS-500 dont la surface spécifique est de 700 - 900 m²/g et la taille de microparticules de 75 - 100 µm.
